Europäisches Patentamt

European Patent Office

Office européen des brevets

(11) Veröffentlichungsnummer: **0 372 230 B1**

## EUROPÄISCHE PATENTSCHRIFT

(45) Veröffentlichungstag der Patentschrift: **26.05.93**

(51) Int. Cl.5: **C12N 15/70**, C12N 15/77, C12N 15/00

(21) Anmeldenummer: **89120473.7**

(22) Anmeldetag: **06.11.89**

(54) **Verfahren zum konjugativen Transfer von mobilisierbaren Vektoren aus E.coli in Gram-positive Bakterien und dafür geeignete Vektoren.**

(30) Priorität: **09.12.88 DE 3841453**

(43) Veröffentlichungstag der Anmeldung:
**13.06.90 Patentblatt 90/24**

(45) Bekanntmachung des Hinweises auf die
Patenterteilung:
**26.05.93 Patentblatt 93/21**

(84) Benannte Vertragsstaaten:
**BE DE ES FR GB IT NL**

(56) Entgegenhaltungen:

**CHEMICAL ABSTRACTS, Band 108, Nr. 7, 15. Februar 1988, Seiten 210-211, Zusammenfassung Nr. 50596t, Columbus, Ohio, US; P. TRIEU-CUOT et al.: "Plasmid transfer by conjugation from Escherichia coli to Gram-positive bacteria", & FEMS MICROBIOL. LETT. 1987, 48(1-2), 289-94**

**PATENT ABSTRACTS OF JAPAN, Band 10, Nr. 43 (C-329)[2100], 20. Februar 1986; & JP-A-60 192 586 (KYOWA HAKKO KOGYO K.K.) 01-10-1985**

(73) Patentinhaber: **Degussa Aktiengesellschaft**
**Weissfrauenstrasse 9**
**W-6000 Frankturt am Main 1(DE)**

(72) Erfinder: **Schäfer, Andreas**
**Bünder Strasse 33**
**W-4800 Bielefeld 1(DE)**
Erfinder: **Kalinowski, Jörn**
**Drögestrasse 25**
**W-4800 Bielefeld 1(DE)**
Erfinder: **Pühler, Alfred, Prof.**
**Am Waldschlösschen 2**
**W-4800 Bielefeld 15(DE)**

EP 0 372 230 B1

## Beschreibung

Die Erfindung betrifft ein Verfahren zum konjugativen Transfer von mobilisierbaren Vektoren aus E.coli in Gram − positive Bakerien und dafür geeignete Vektoren.

Der Einsatz von Transposons zur Mutagenese, Genisolierung und − Analyse war bislang weitgehend auf Gram − negative Bakterien beschränkt. Als Ausnahme kann in diesem Zusammenhang die Transposonmutagenese von Bacillus subtilis durch das Transposon Tn917 eingestuft werden (Youngman et al. PNAS80, 4 (1983) 2305 − 2309), die dadurch ermöglicht wird, daß B.subtilis über eine ausreichend hohe natürliche Kompetenz zur Aufnahme von Plasmid − DNA aus dem umgebenden Medium verfügt.

P.Trieu − Cuot et al. (FEMS Microbiology Letters 48 (1987) 289 − 294) beschreiben zwar den Plasmid − transfer von E.coli in bestimmte Gram − positive Bakterien durch Konjugation, erzielen aber mit Transfer − frequenzen von $10^{-7}$ bis $10^{-8}$ nur sehr unbefriedigende Werte.

Eigene Versuche zeigen zudem, daß mit dem dort beschriebenen System ein Transfer in Corynebacterium glutamicum nicht nachzuweisen ist.

Aus den US − PSen 4,626,506, 4,680,264 und 4,686,184 (Pühler et al.) und durch Simon et al. (Biotechnology, November 1983 und Methods in Enzymology, Vol. 118, 640 − 659) ist die Mutagenese in Gram − negativen Bakterien mit Hilfe von mobilisierbaren E.coli Vektoren bekannt.

Aufgabe der Erfindung ist die Entwicklung einer Kreuzungsvorschrift (konjugativer Transfer), nach der mobilisierbare Vektorplasmide in hoher Frequenz aus E.coli in Gram − positive, insbesondere coryneforme Bakterien transferiert werden können.

Gegenstand der Erfindung ist ein Verfahren zum konjugativen Transfer von mobilisierbaren Vektoren aus E.coli in Gram − positive Bakterien, das dadurch gekennzeichnet ist, daß man restriktionsdefekte Zellen eines Gram − positiven Bakteriums herstellt und diese nach an sich bekannten Kreuzungsverfahren mit einem den mobilisierbaren Vektor tragenden E.coli Mobilisatorstamm mischt.

Während sich der Donor bevorzugt in der logarithmischen Wachstumsphase befindet, hat sich für den Zustand des Rezipienten die stationäre Wachstumsphase als günstig erwiesen.
Donor − und Rezipientenzellen werden im allgemein im Verhältnis 1 : 2 bis 1 : 10, bevorzugt 1 : 4 bis 1 :6, insbesondere 1 : 5 eingesetzt.

Die geeigneten mobilisierbaren E.coli Vektoren sind nicht selbsttransferierbar.

Der Begriff E.coli Vektoren beinhaltet allgemein alle nur in E.coli − Stämmen selbständig replizierenden Plasmide, die sich nach dem Stand der Technik als für gentechnologische Anwendungen nützlich erwiesen haben.

Beispiele solcher E.coli Vektoren sind
pMB9, pBR322, pBR325, pKB111, pUC8, pUC9, pACYC184, pACYC177, pSC101.

Übliche E.coli Vektoren wir pBR325 (Bolivar, F. et al., Gene 2, 95, (1977) oder pACYC184 (Chang, AC.Y. und Cohen, S.N., J. Bact. 134, 1141 (1978) sind weder selbsttransferierbar noch ausreichend mobilisierbar.

Diese und andere Vektoren, die nur in Bakterienstämmen der E.coli − Gruppe replizieren, werden durch Insertion der Mob − site eines Plasmides mit weitem Wirtsbereich in Gram − negativen Bakterien modifiziert.

Bevorzugt wird das Plasmid RP4 für diese Zwecke verwendet. E.coli − Vektoren, die ein 1,9 kb großes Fragment (Mob − site) von RP4 tragen, lassen sich in dem erfindungsgemäßen Verfahren vorteilhaft einsetzen.

Als Mobilisatorstämme geeignet sind modifizierte E.coli − Stämme, die ein Plasmid im Chromosom integriert oder frei vorliegend enthalten, das in der Lage ist, die zur Mobilisierung notwendigen Funktionen bereitzustellen.

Es eignen sich insbesondere Stämme, in deren Chromosom ein RP4 − Derivat integriert ist, dessen Transfer − Funktion intrans auf die Mob − site der oben genannten Vektoren einwirkt.

Geeignete Vektoren und E.coli Mobilisatorstämme sind aus der US − PS 4,626,504 bekannt und beim Northern Regional Research Center hinterlegt:

| Stämme | | NRRL-Hinterlegungsnummer |
|---|---|---|
| E.coli | CSH52/pSUP101 | B - 15484 |
| E.coli | CSH52/pSUP201 | B - 15487 |
| E.coli | CSH52/pSUP202 | B - 15488 |
| E.coli | CSH52/pSUP203 | B - 15489 |
| E.coli | CSH52/pSUP301 | B - 15492 |
| E.coli | CSH52/pSUP401 | B - 15494 |
| E.coli | SM10 | B - 15481 |
| E.coli | S68-7 | B - 15482 |
| E.coli | S17-1 | B - 15483 |

Tabelle 1

Weitere Vektoren, wie pSUP102 oder pSUP205 sind aus der Literatur bekannt und werden nach analogen Verfahren aus bekannten Vektoren gewonnen (Simon et al., Methods of Enzymology 118, 640 ff (1986) und Biotechnology, November 1983).

Der Restriktionsdefekt kann genetisch bedingt sein und z. B. durch mutagene Agentien (z.B. NTG:Methylnitro – nitrosoguanidin), erzeugt werden, er kann aber auch physiologisch bedingt sein, z. B. durch einen Hitzeschock.

Als besonders effektiv hat sich die Hitzebehandlung des Rezipienten unmittelbar vor der Kreuzung erwiesen. Dabei sind intakte oder sphäroplastierte Zellen einzusetzen.

Ein Hitzeschock für die Dauer von 1 bis 30 min, bevorzugt ca. 9 min, bei 45 bis 55 °C, bevorzugt ca. 49 °C, ermöglicht es, mit der sich daran anschließenden Steigerung der Transferfrequenz die Aufgabe der Erfindung zu erfüllen.

Vektoren, wie sie in Tabelle 1 aufgeführt sind, sind auch als Suizid – Vektoren zu bezeichnen, da sie nach dem Transfer in Gram – positive Bakterienstämme dort nicht mehr replizieren können.

Sie eignen sich jedoch zur Insertionsmutagenese von Gram – positiven Bakterienstämmen, indem man ein kloniertes z. B. ein für eine Enzymaktivität in einem Gram – positiven Bakterienstamm codierendes Gen(fragment) in einen der erfindungsgemäß verwendbaren mobilisierbaren Vektoren nach bekannten Verfahren inseriert und den resultierenden Vektor durch Konjugation von einem E.coli Mobilisatorstamm in einen Gram – positiven Stamm transferiert, in dem eine homologe Rekombination mit dem dort befindlichen entsprechenden intakten Gen erfolgen kann.

Die homologe Rekombination führt entweder zur Insertion des gesamten resultierenden Vektors (single cross – over) oder zu einem Austausch des eingeschleusten Genfragments gegen die homologe Sequenz im intakten Gen des Rezipienten (double cross – over).

Das erfindungsgemäße Verfahren wird auch mit mobilisierbaren, nicht selbsttransferierbaren Vektoren durchgeführt, die zusammengesetzt sind aus

a) einem DNA – Segment, enthaltend ein in E.coli funktionelles Replikon,
b) einem zweiten DNA – Segment, enthaltend die für die Mobilisierungs – und die Transferfunktion codierenden DNA – Fragmente (Mob – site und ori T),
c) einem dritten DNA – Segment, das sich von einem in coryneformen Bakterien, insbesondere Coryne – bacterium glutamicum replizierendem Vektor ableitet, und
d) gegebenenfalls einem Transposon anstelle von (c) oder in (c) enthalten.

Ein erfindungsgemäßer Vektor ist insbesondere pECM1 (DSM 4982), dessen Hinterlegung bei der Deutschen Sammlung für Mikroorganismen (DSM) nach dem Budapester Vertrag erfolgte.

In diesem Zusammenhang ist darauf hinzuweisen, daß der Begriff Vektor in diesem Text je nach Herkunft des zu bezeichnenden Elements für Plasmidvektor, Phagenvektor oder Plasmid steht.

Erleichtert wird auch das Auffinden und Verkleinern von Replikons als Basis der Vektorentwicklung für Gram – positive Bakterien.

Das Kreuzungsprotokoll erlaubt den konjugativen Transfer mobilisierbarer Shuttle – Vektoren aus einem E.coli – mobilisatorstamm in zellwandintakte oder sphäroplastierte Gram – positive Bakterien mit einer Häu –

figkeit von ca. $10^{-1}$ bis $10^{-3}$, bezogen auf den Donortiter.

Die Übertragung der Vektoren kann anhand charakteristischer Antibiotika – Resistenzmarker, sowie durch Lyse der Transkonjuganten mit anschließender Analyse des Plasmidgehaltes durch Agarose – Gelektrophorese überprüft werden. Eine Veränderung der Vektoren als Resultat des Konjugationsprozesses wird nicht registriert. Natürliche Transformation als Übertragunsmechanismus kann durch Kontrollkreuzun – gen ausgeschlossen werden.

Die mittels konjugativen Transfer erzielten hohen Transfersequenzen ermöglichen zukünftig einen sinnvollen Einsatz von Transposons auch bei solchen Gram – positiven Bakterien, für die ein natürliches Transformationssystem bislang nicht demonstriert werden konnte.

Bevorzugt sind weitere Vektoren, bei denen sich die DNA – Segmente (a) und (b) von einem der Vektoren aus der Gruppe pSUP101, pSUP102, pSUP201, pSUP202, pSUP203, pSUP205, pSUP301, pSUP401 ableiten.

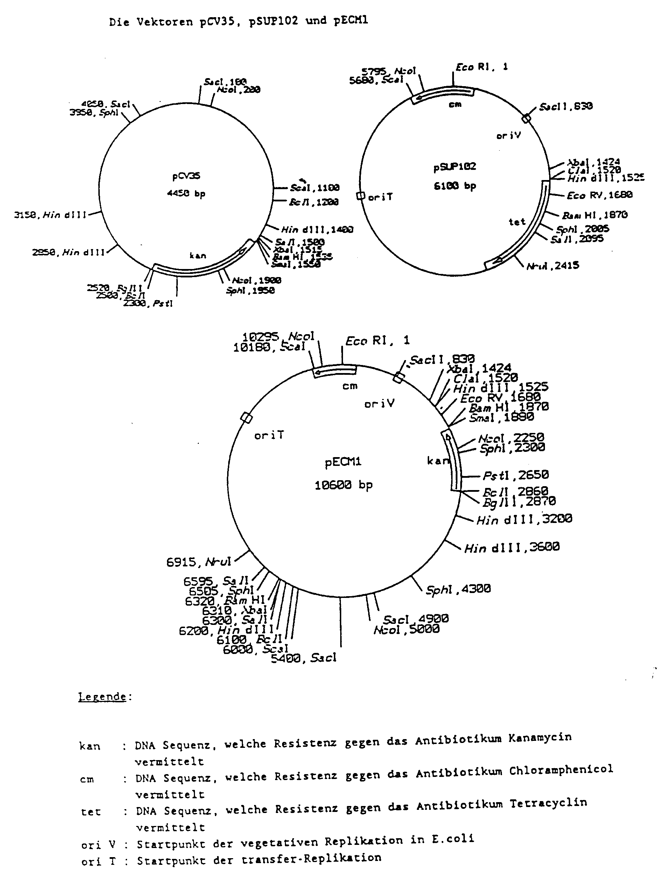

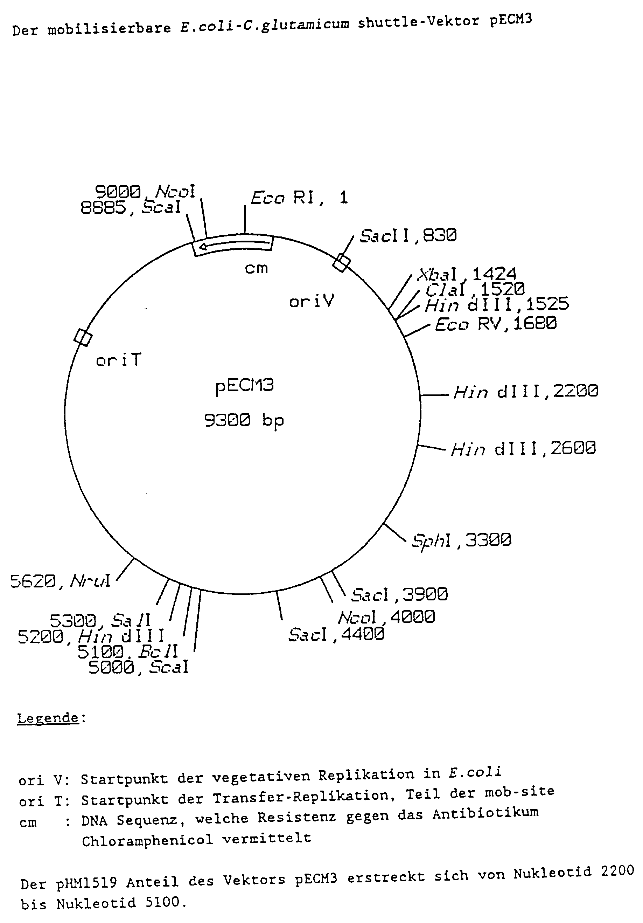

Vektoren, die diese bevorzugten Merkmale aufweisen, sind pECM1 und pECM3, deren Restriktionskar – ten in den Abb. 1 bzw. 2 wiedergegeben werden.

Der Wirtsbereich dieser Vektoren umfaßt die Aminosäurenausscheidenden Stämme der Gattungen Cornyebacterium und Brevibacterium, insbesonders
Corynebacterium acetoacidophilum
Corynebacterium glutamicum
Corynebacterium glutamicum ATCC 13032
Corynebacterium glutamicum ATCC 13058
Corynebacterium hyfrocarboclastum
Cornyebacterium ilicis
Corynebacterium lilium
Corynebacterium sp. DSM 20140
Brevibacterium flavum
Brevibacterium lactofermentum
Brevibacterium lyticum
Brevibacterium roseum
Micrococcus sodonense
Das erfindungsgemäße Verfahren zum konjugativen Transfer ermöglicht auch die schnelle Bestimmung des Wirtbereichs von Replikons, insbesondere von bekannten Plasmiden, wie z. B. pHM1519.

Beispiel 1

Konstruktion eines mobilisierbaren Shuttle – Vektors

Der mobilisierbare Shuttle – Vektor pECM1 (Abb.1) wurde konstruiert durch Fusion des mobilisierbaren *E.coli* Vektors pSUP102 (Abb.1; Simon et al. (1)) mit dem *Corynebakterium glutamicum* – Plasmid pCV35 (Abb.1;)

0,5µg pSUP102 DNA und 1µg pCV35 DNA werden im Spaltungspuffer React 3 (Hersteller: BRL – Gibco, Karlsruhe), in Gegenwart von je 1u BamHI – Restriktionsenzym (BRL – Gibco, Karlsruhe), für 1h bei 37˚C inkubiert.

Vollständig gespaltene pSUP102 DNA wird im Spaltungspuffer mit der linearisierten pCV35 DNA versetzt und das Gemisch wird für 5 min bei 70˚C inkubiert. Mit Hilfe des Ligationspuffers (BRL – Gibco) und ATP werden die Bedingungen für die Ligation eingestellt. In Gegenwart von 1u T4 DNA – Ligase wird für 16h bei 14˚C ligiert.

Das Ligationsgemisch wird durch Transformation (nach Maniatis et al. (5)) in Zellen des Stammes *E.coli* S17 – 1 eingebracht, welche infolge $CaCl_2$ – Behandlung zur Aufnahme von Plasmid – DNA befähigt sind.

Zellen, die pECM1 erhalten, werden resistent gegen die Antibiotika Kanamycin und Chloramphenicol (50µg/ml), sind aber sensitiv gegen Tetracyclin (5µg/ml).

pECM1 repliziert in *E.coli* und in *C.glutamicum* und ist mit Hilfe der transfer – Funktionen des im Chromosom des Mobilisatorstammes *E.coli* S17 – 1 integrierten RP4 – Derivates mobilisierbar.

pECM1 ist in E.coli S17 – 1 hinterlegt bei der Deutschen Sammlung für Mikroorganismen unter der Nr. DSM 4982.

Mutagenese der BglII – Schnittstelle von pCV34 und Erzeugung des Plasmids pCV35

Das Plasmid pCV34, hinterlegt als DSM 5025, wurde nach Thierbach et al. (1988) (6) isoliert und mit dem Enzym BglII gespalten. Der Mutageneseansatz besteht aus etwa 2 $\mu$g Plasmid – DNA in 60 $\mu$l TE – Puffer, 180 $\mu$l 1.5M Hydroxylamin – HCl in 25 mM EDTA, 5 $\mu$l 0,25 M EDTA und 13 ul 1M Tris – HCl, pH 8.0. Der Ansatz wurde gemischt und 20 Minuten bei 60 ˚C inkubiert. Nach Phenol – Behandlung und Äthanol – Fällung der Plasmid – DNA (Maniatis et al. 1982) (5) wurde das DNA – Pellet in 20 ul TE aufgenommen und mit T4 – DNA – Ligase behandelt. Nach Transformation von C.glutamicum ATCC 13032 mit der Plasmid – DNA konnten Transformanten isoliert werden, die eine der beiden BglII – Schnittstellen verloren hat. pCV35 ist neu und weist die gleiche Kanamycin – Resistenzhöhe auf wie pcV33 (s. parallele Anmeldung DE – 38 41454)

Konstruktion des Shuttle Vektors pECM3

pECM3 (Abb. 2) entsteht aus pECM1 durch Deletion eines 0,3 kb großen SalI – Fragmentes und eines BamHI/BglII – Fragmentes, welches die Information für die Kanamycin – Resistenz trägt.

Die Konstruktion dieses Vektors erfolgt nach allgemein bekannten Verfahren aus dem hinterlegten Vektor pECM1.

Beispiel 2

Detailliertes Kreuzungsprotokoll

Lediglich zur Vereinfachung wird im folgenden die Erfindung anhand der Mobilisierung des Shuttle – Vektors pECM1 aus *E.coli* S17 – 1 nach *C.glutamicum* ATCC 13032 beschrieben. Bei Verwendung entsprechender Replicons ergibt sich jedoch eine Übertragbarkeit auch auf andere Mikroorganismen (siehe hierzu auch Beispiel 3).

Für einen konjugativen Transfer von pECM1 aus *E.coli* S17 – 1 in zellwandintakte *C.glutamicum* ATCC 13032 – Zellen wird folgende Kreuzungsvorschrift verwendet:

10ml Luria Broth Medium met 50$\mu$g/ml Kanamycin werden im Reagenzglas mit pECM1 tragenden Zellen des Mobilisatorstammes *E.coli* S17 – 1 beimpft und über Nacht bei 37˚C im Roller inkubiert (Donor – Vorkultur).

Für die Anzucht des Rezipienten werden pro Kreuzungsansatz 10ml Luria Broth – Glukose Medium (mit 50$\mu$g/ml Nalidixinsäure) im Reagenzglas mit *C.glutamicum* ATCC 13032 beimpft und bei 30˚C im Roller inkubiert, bis eine optische Dichte von 3 – 4, bei einer Wellenlänge von 580nm, erreicht ist (ca.20h).

Für die Kreuzung werden pro Ansatz 10ml Luria Broth Medium (mit 50$\mu$g/ml Kanamycin) im Reagenz – glas mit 100$\mu$l der Donor – Vorkultur beimpft und bei 37˚C im Roller inkubiert, bis eine optische Dichte (bei 580nm) von 0,6 – 0,7 erreicht ist.

Die Rezipientenkulturen werden im Reagenzglas für 13min in einem Wasserbad einer Temperatur von 49˚C ausgesetzt (Hitzeschock). Die hitzebehandelten Rezipientenkulturen werden in Sterile PE – Röhrchen überführt und für 8min bei 3000 U/min abzentrifugiert. Der Überstand wird verworfen und das Pellet wird im Rücklauf resuspendiert.

Für die Kreuzung werden Donor – und Rezipientenzellen im Verhältnis 1:5 eingesetzt. Eine entspre – chende Menge an Donorkultur wird in sterile PE – Röhrchen überführt und für 8min bei 3000 U/min abzentrifugiert. Der Überstand wird vollständig verworfen.

Anschließend werden die resuspendierten Rezipientenzellen zum Donorpellet pipettiert. Die Suspension wird erneut für 8min bei 3000 U/min zentrifugiert und der Überstand auf maximal 500$\mu$l reduziert. Die Zellen werden sehr vorsichtig resuspendiert und auf Celluloseacetatfilter (0,45$\mu$m Porengröße) aufgetropft, welche auf Luria Broth – Glukose Festmedium aufgelegt wurden.

Die Platten werden 18h bei 30˚C inkubiert und die Filter anschließend mit 0,8ml Luria Broth Medium abgeschwemmt. Die Selektion der Transkonjuganten erfolgt bei 30˚C auf Luria Broth – Glukose Festmedi – um, welches Kanamycin (25$\mu$g/ml) und Nalidixinsäure (50$\mu$g/ml) enthält.

Soll der konjugative Transfer von pECM1 in sphäroplastierte Rezipientenzellen erfolgen, sind folgende Änderungen zu beachten:

Der Sphäroplastenansatz (ca. 330$\mu$l) wird vor Durchführung der Hitzebehandlung in 10ml TSMC* – Puffer suspendiert und für mindestens 3h bei 30˚C inkubiert.

Rezipientenzellen und Kreuzungsgemisch werden nach Zentrifugation nicht in Luria Broth Medium, sondern in TSMC* – Puffer resuspendiert.

Die Selektion der Transkonjuganten erfolgt auf osmotisch stabilisierten Sorbitol(SB) − Regenerationsplatten, welche Kanamycin (17,5$\mu$g/ml und Nalidixinsäure (50$\mu$g/ml enthalten.

Konjugativer Transfer des mobilisierbaren Shuttle − Vektors pECM1 aus *E.coli* S17 − 1 nach *Corynebacterium glutamicum* ATCC 13032

Der mobilisierbare Shuttle − Vektor pECM1 (Beispiel 1) wird, der bereits beschriebenen Kreuzungsvorschrift folgend, aus *E.coli* S17 − 1 nach *C.glutamicum* mobilisiert.

Als Rezipienten werden sowohl sphäroplastierte, als auch zellwandintakte *C. glutamicum* − Zellen verwendet. Zusätzlich wird eine *C.glutamicum* − Mutante eingesetzt, deren Restriktionssystem durch Mutagenese mit NTG ausgeschaltet wurde (res⁻ − Mutante*).

Für den Transfer von pECM1 durch Konjugation ergeben sich folgende Frequenzen:

| Rezipient | nicht sphäropl. res$^+$ − Zellen | sphäropl. res$^+$ − Zellen | nicht sphäropl. res⁻ − Zellen |
|---|---|---|---|
| ohne Hitzeschock | $5 \times 10^{-7}$ | $3 \times 10^{-7}$ | $2 \times 10^{-2}$ |
| mit Hitzeschock | $1 \times 10^{-2}$ | $4 \times 10^{-3}$ | $9 \times 10^{-3}$ |

Die ermittelten Frequenzen sind Mittelwerte dreier unabhängig voneinander durchgeführter Kreuzungen und beziehen sich jeweils auf den Donortiter.

Das hier aufgeführte Beispiel zeigt deutlich, da eine zeitlich limitierte Hitzebehandlung (13min,49˚C), das Restriktionssystem der Rezipientenzellen beeinträchtigt und zu um den Faktor $10^4 − 10^5$ erhöhten Transferfrequenzen führt.

Ein effizienter Transfer von pECM1 mit Hilfe eines Hitzeschocks ist sowohl in zellwandintakte, als auch in sphäroplastierte *C.glutamicum* − Zellen möglich. Der zu beobachtende geringe Unterschied in den Transferfrequenzen ist Ausdruck einer durch die Schädigung der Zellwand erhöhten Tendenz der sphäroplastierten Zellen zur Lyse.

Die bei der Verwendung hitzebehandelter Rezipienten erzielten Transferfrequenzen werden ohne Durchführung einer Hitzebehandlung nur bei Einsatz einer, für *C.glutamicum* zur Verfügung stehenden, restriktionsdefekten Mutante (res⁻ − Mutante*) erreicht.

Beispiel 3

Konjugativer Transfer von pECM1 in coryneforme Bakterien − Bestimmung des Wirtsbereiches von pHM1519

pHM1519 ist ein 3kb großes, kryptisches Plasmid, welches aus *C.glutamicum* ATCC 13058 isoliert wurde (Miwa et al. (7)).

pHM1519 ist das Basisreplikon des *C.glutamicum* − Vektors pCV35, welcher für die Konstruktion des mobilisierbaren *E.coli− C.glutamicum* Shuttle − Vektors pECM1 (Beispiel 1) verwendet wurde.

Für die Bestimmung des Wirtsbereiches von pHM1519 wird pECM1 aus *E.coli* S17 − 1 in Vertreter aus der Gruppe der coryneformen Bakterien mobilisiert.

Die Kreuzungen werden nach der bereits beschriebenen Vorschrift durchgeführt. Die Selektion der Transkonjuganten erfolgt auf LBKm$_{25}$Nx$_{50}$ − Medium, wobei die innerhalb der coryneformen Bakterien verbreitete natürliche Resistenz gegen das Antibiotikum Nalidixinsäure zur Selektion gegen den Donor ausgenutzt wird.

Der Transfer von pECM1 wird anhand der durch den Vektor vermittelten Resistenzen gegen die Antibiotika Kanamycin und Chloramphenicol, sowie durch Lyse der Transkonjuganten (nach Birnboim et al. (8), modifiziert) und anschließender Analyse des Plasmidgehaltes durch Agarose − Gelektrophorese verifiziert.

Das Resultat dieses Experimentes zeigt, das der Wirtsbereich von pHM1519 eine Reihe von Vertretern aus der Gruppe der coryneformen Bakterien umfaßt, die z.T. von erheblichem kommerziellen Interesse sind:
*Corynebacterium acetoacidophilum*
*Corynebacterium glutamicum* ATCC 13032
*Corynebacterium glutamicum* ATCC 13058
Corynebacterium hydrocarboclastum
Corynebacterium ilicis
Corynebacterium lilium
Corynebacterium sp. DSM 20140
Brevibacterium divaricatum

Brevibacterium flavum
Brevibacterium lactofermentum
Brevibacterium lyticum
Brevibacterium roseum
Brevibacterium stationis
Micrococcus sodonense
Corynebacterium callunae DSM 20147
Corynebacterium pilosum DSM 20521
Corynebacterium fascians DSM 20131
Corynebacterium herculis DSM 20301
Corynebacterium melassecola ATCC 17965
Corynebacterium melassecola ATCC 17966
Arthrobacter albidus DSM 20128
Brevibacterium ammoniagenes 20305

Gleichartige Tests ergaben, daß folgende Stämme nicht zum Wirtsbereich des pHM1519 Replicons gehören:
Clavibacter michiganenense
Clavibacter nebraskense
Corynebacterium flaccumfaciens
Bacillus subtilis
Brevibacterium linens
Brevibacterium ketoglutamicum
Brevibacterium pusillum
Brevibacterium testaceum

Beispiel 4

Insertionsmutagenese von *C.glutamicum* am Beispiel des *lysA* – Gens

Das *lysA* – Gen kodiert für das Enzym meso – Diaminopimelat – Decarboxylase, das den letzten Schritt in der Lysinbiosynthese katalysiert. Nach Komplementation der *E.coli lysA* – Mutante W7 (Wientjes et al. (9)) mit einer im *E.coli* – Vektor pUC18 angelegten Genbank von *C.glutamicum* ATCC 13032, die in der europäischen Patentanmeldung 89114632.6 beschrieben ist, wurde das Hybridplasmid pTG1225 isoliert, das eine 5,8 kb lange Insertion von ATCC 13032 – DNA trägt.

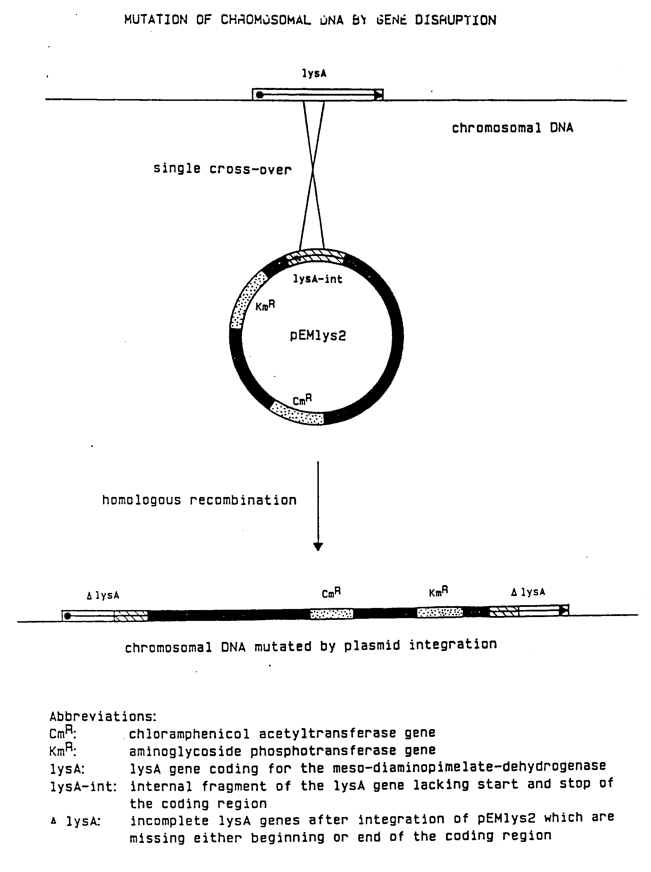

Anhand der veröffentlichten Sequenz des *lysA* – Gens von *C.glutamicum* (Yeh et al. (10) ) wurde ein 1 kb langes *Bcl*I – *Sal*I – Fragment des Plasmids pTG1225 gewählt, das eine interne Region der *lysA* – Kodier – region trägt. Bei homologer Rekombination in das Chromosom wird das chromosomale Gen durch Integration des Vektors inaktiviert (Abb. 3).

Zur Mutagenese durch Integration wurde der mobilisierbare, nicht selbsttransmissible Vektor pEMlys2 konstruiert, der zusammengesetzt ist aus

a) dem Plasmid pSUP102 (Simon et al. (1)), das ein in *E.coli* funktionelles Replikon und die Mob – Region enthält,

b) einem internen Fragment des *lysA* – Gens aus *C.glutamicum*, und

c) der in *C.glutamicum* selektionierbaren Kanamycin – Resistenzdeterminante des Transposons Tn*903* (Veira & Messing (11)).

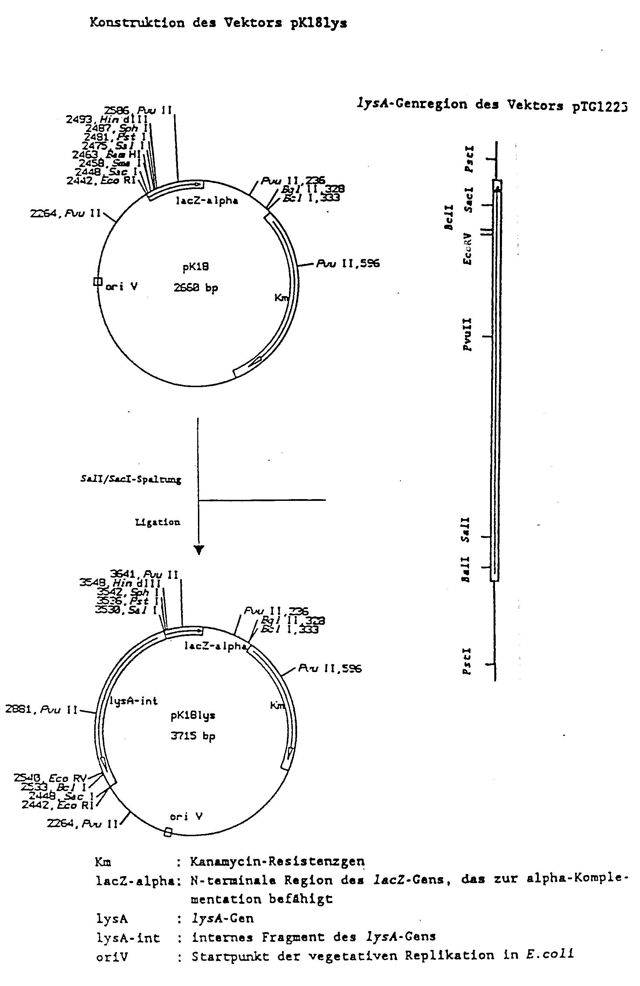

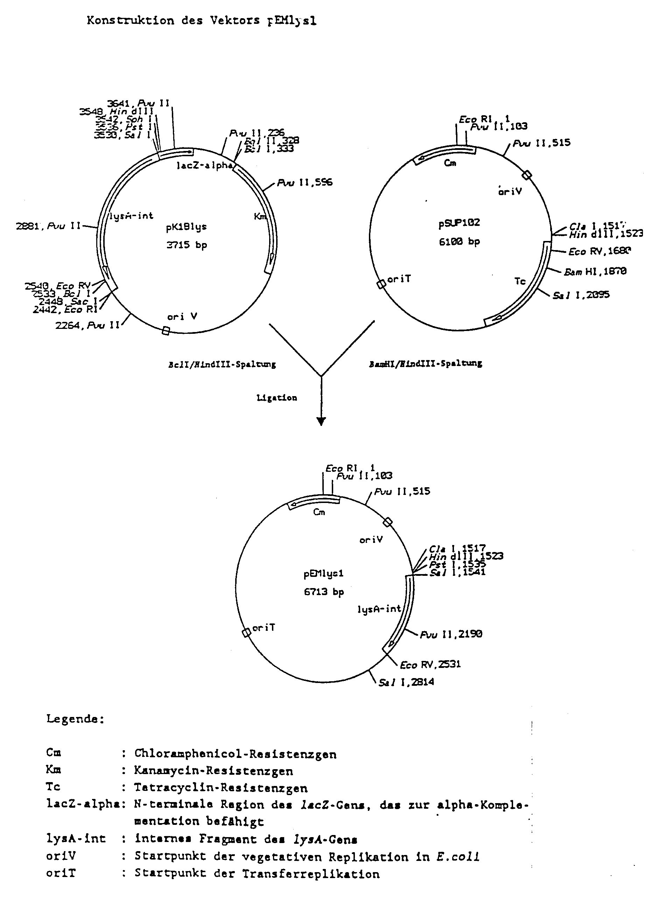

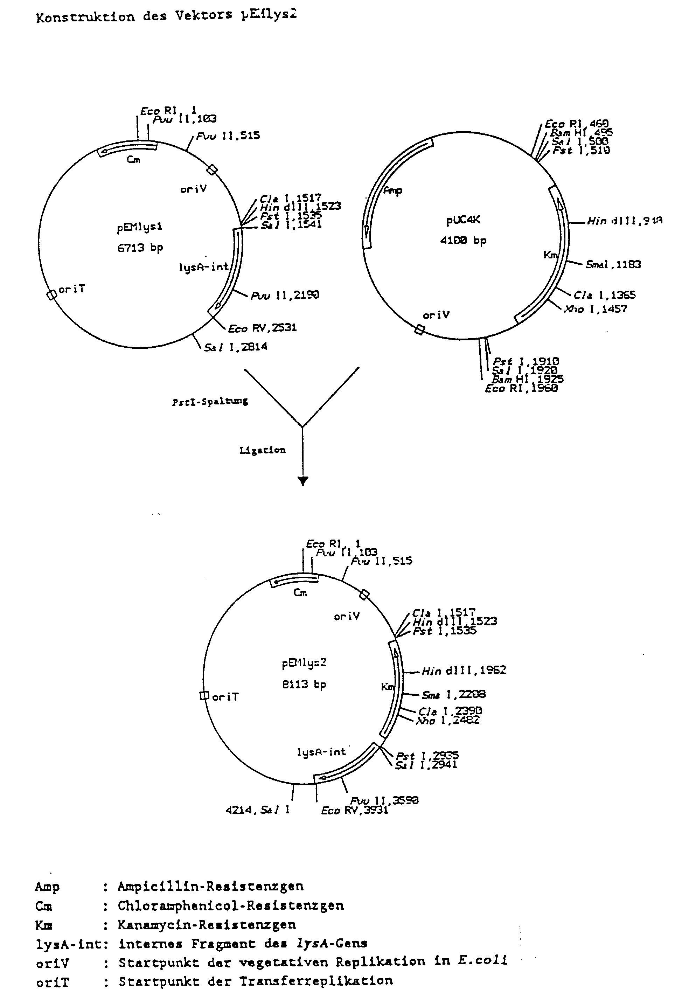

Die Konstruktion des Vektors pEMlys2 (Abb.4 – 6) erfolgte nach den bekannten Methoden der rekom – binanten DNA – Technologie. Ein internes Fragment des *lysA* – Gens (1,1 kb) wurde mittels der Restrik – tionsenzyme *Sal*I und *Sac*I aus dem Plasmid pTG1225 herausgeschnitten und mit dem ebenso gespaltenen Vektor pK18 (Pridmore (12) ) ligiert.

Aus dem resultierenden Plasmid pK18lys (3,7 kb) wurde durch Spaltung mit den Restriktionsenzymen *Bcl*I und *Hind*III ein internes Fragment der *lysA* – Kodierregion isoliert und mit dem *Bam*HI und *Hind*III restringierten Vektor pSUP102 fusioniert.

In den resultierenden Vektor pEMlys1 (6,7 kb) wurde das Kanamycin – Resistenzgen des Transposons Tn*903* kloniert. Hierzu wurde pEMlys1 durch Verdauung mit *Pst*I linearisiert und mit einem 1,4 kb langen *Pst*I – Fragment des Vektors pUC4K ligiert.

Der so entstandene Vektor pEMlys2 (8,1 kb) wurde in den Mobilisatorstamm *E.coli* S 17 – 1 transfor – miert. Die Konjugation dieses Plasmides von *E.coli* nach *C.glutamicum* ATCC 13032 erfolgte nach dem in Beispiel 2 detailliert dargestellten Protokoll. Kanamycin – resistente Kolonien traten mit einer Frequenz von 2

EP 0 372 230 B1

x $10^{-7}$ bezogen auf den Donortiter auf.

Die Kanamycin−resistenten Transkonjuganten wurden auf Minimalagar (Kaneko & Sakaguchi (13)) und auf mit L−Lysin supplementiertem Minimalagar überstempelt. Die auf diese Weise untersuchten Transkonjug− anten wiesen entsprechend der Insertionsinaktivierung des chromosomalen *lysA*−Gens durch Integration von pEMlys2 einen Lysin−auxotrophen Phänotyp auf.

Bei einer nach Birnboim et al. (8) durchgeführten Lyse der Transkonjuganten mit anschließender Analyse des Plasmidgehalts durch Agarose−Gelelektrophorese konnten keine freien Plasmide in den Zellen nachgewiesen werden.

Den Nachweis der spezifischen Integration ergab eine Digoxygenin−Hybridisierung (14) des markierten *E.coli*−Vektors pSUP102 gegen Gesamt−DNA isoliert aus *C.glutamicum*−Wildtypzellen und den Transkonjuganten, wobei nur DNA aus Transkonjuganten mit der DNA des *E.coli*−Vektors hybridisierte.

Literatur

(1) R. Simon et al.
Plasmid Vectors for the Genetik Analysis and Manipulation of Rhizobia and Other Gram−Negative Bacteria
Methods in Enzymology, Vol. 118, 1986 S.641−658
(2) R. Simon, U.Priefer, A.Pühler
A broad host range mobilization system for in vivo genetic engineering: Transposon mutagenesis in gram−negative bacteria
Biotechnology 1, 784−794 (1983).
(3) Youngman et al. PNAS 80, 4/1983, S.2305−2309
(4) P. Trien−Cuot et al. (FEMS Microbiology Letters, 48 (1987) 289−294.
(5) Maniatis et al.
Molecular cloning, Cold Spring Harbor Laboratory Publications 1982
(6) G.Thierbach, A.Schwarzer, A.Pühler
Transformation of sphaeroplasts and protoplasts of *C. glutamicum*
Appl. Microbiol. Biotechnol. 29 (1988), 356−362
(7) K. Miwa et al.
Cryptic plasmids in glutamic acid−producing bacteria
Agric. Biol. Chem. 48, 2901−2903 (1984)
(8) Birnboim et al.
Nucl. Acids Res. 7: 1513−1523
(9) F.B. Wientjes, E. Pas, P.E.M. Taschner & C.L. Woldringh
Kinetics of uptake and incorporation of meso−diaminopimelic acid in different *Escherichia coli* strains
J. Bacteriol. 164 (1985): 331−337
(10) P.Yeh, A.M. Sicard & A.J. Sinskey
Nucleotide sequence of the *lysA* gene of *Corynebacterium glutamicum* and possible mechanisms for modulation of its expression
Mol. Gen. Genet. 212 (1988): 112−119
(11) J. Veira & J. Messing
The pUC plasmids, an M13mp7−derived system for insertion mutagenesis and sequencing with synthetic universal primers
Gene 19 (1982): 259−268
(12) R.D. Pridmore
New and versatile cloning vectors with kanamycin−resistance marker
Gene 56 (1987): 309−312
(13) H. Kaneko & K. Sakaguchi
Fusion of protoplasts and genetic recombination of *Brevibacterium flavum*
Agric. Biol. Chem. 43 (1979): 1007−1013
(14) E.W. Khandjian
Bio/Technology 5 (1987): 165

**Patentansprüche**

1. Verfahren zum konjugativen Transfer von mobilisierbaren Vektoren aus E.coli Mobilisatorstämmen in Gram−positive Bakterien,

8

dadurch gekennzeichnet, daß man restriktionsdefekte Zellen eines Gram – positiven Bakteriums herstellt und diese nach an sich bekannten Verfahren mit einem den mobilisierbaren Vektor tragenden E.coli Stamm kreuzt.

2. Verfahren gemäß Anspruch 1,
dadurch gekennzeichnet, daß der Vektor eine DNA – Region (Mob – site) und der E.coli Mobilisator – stamm ein Plasmid im Chromosom integriert oder frei vorliegend enthält, das in der Lage ist, die zur Mobilisierung notwendigen Funktionen bereitzustellen.

3. Verfahren gemäß Anspruch 2,
dadurch gekennzeichnet, daß der Vektor ein 1,9 kb großes DNA – Fragment (Mob – site) des Plasmids RP4 trägt und in das Chromosom des E.coli Mobilisatorstammes ein RP4 – Derivat integriert ist, dessen transfer Funktion in trans auf die Mob – site des genannten Vektors wirkt.

4. Verfahren gemäß Anspruch 3,
dadurch gekennzeichnet, daß man den E.coli – Stamm aus der Gruppe E.coli S17 – 1, E.coli SM10 oder E.coli S68 – 7 auswählt.

5. Verfahren gemäß einem oder mehreren der Ansprüche 2 bis 4, dadurch gekennzeichnet, daß man den E.coli Vektor aus der Gruppe pSUP101, pSUP102, pSUP201, pSUP202, pSUP203, pSUP205, pSUP301, pSUP401 auswählt.

6. Verfahren gemäß einem oder mehreren der Ansprüche 1 bis 5, dadurch gekennzeichnet, daß man das Restriktionssystem des einzusetzenden Gram – positiven Bakteriums durch einen Hitzeschock unmit – telbar vor der Kreuzung beeinträchtigt.

7. Verfahren gemäß einem oder mehreren der Ansprüche 1 bis 6, dadurch gekennzeichnet, daß man intakte Zellen des Rezipienten einsetzt.

8. Verfahren gemäß einem oder mehreren der Ansprüche 1 bis 6, dadurch gekennzeichnet, daß man sphäroplastierte Zellen des Rezipienten einsetzt.

9. Verfahren gemäß einem oder mehreren der Ansprüche 1 bis 8, dadurch gekennzeichnet, daß man einen mobilisierbaren Vektor einsetzt, in den ein in einem Gram – positiven Bakterium aktives Gen – (fragment) inseriert ist, das in dem zu transformierenden Bakterium homolog mit dem entsprechenden Gen rekombiniert.

10. Verfahren gemäß einem oder mehreren der Ansprüche 1 bis 8, dadurch gekennzeichnet, daß man einen mobilisierbaren Vektor einsetzt, in den ein Transposon und gegebenenfalls ein für eine Enzym – aktivität in E.coli oder einem Gram – positiven Bakterium codierendes Gen(fragment) inseriert ist.

11. Verfahren gemäß einem oder mehreren der Ansprüche 1 bis 8, dadurch gekennzeichnet, daß man einen mobilisierbaren Vektor einsetzt, der mit einem in dem Rezipienten replizierenden Plasmid oder Vektor zu einem Shuttle – Vektor fusioniert ist.

12. Mobilisierbarer, nicht selbsttransferierbarer Vektor, zusammengesetzt aus
a) einem DNA – Segment, enthaltend in ein E. coli funktionelles Replikon,
b) einem zweiten DNA – Segment, enthaltend die für die Mobilisierungs – und die Transferfunktion codierenden DNA – Fragmente (Mob – site und ori T),
c) einem dritten DNA – Segment, das sich von einem in coryneformen Bakterien, insbesondere Corynebacterium glutamicum replizierendem Vektor ableitet, und
d) gegebenenfalls einem Transposon anstelle von (c) oder in (c) enthalten.

13. Vektor gemäß Anspruch 12,
dadurch gekennzeichnet, daß sich das dritte DNA – Segment (c) von dem Vektor pCV35 ableitet, dargestellt innerhalb der Abb. 1.

**14.** Vektor gemäß einem oder mehreren der Ansprüche 12 bis 13,
dadurch gekennzeichnet, daß sich die DNA – Segmente (a) und (b) von einem der Vektoren aus der Gruppe pSUP101, pSUP102, pSUP201, pSUP202, pSUP203, pSUP205, pSUP301 oder pSUP401 ableiten.

**15.** Vektor pECM1 (Shuttle – Vektor) gemäß den Ansprüchen 12 bis 14,
gekennzeichnet durch die in Abb. 1 wiedergegebene Restriktionskarte, hinterlegt in E.coli S17 – 1, DSM 4982.

**16.** Vektor pECM3 gemäß den Ansprüchen 12 bis 14,
gekennzeichnet durch die in Abb. 2 wiedergegebene Restriktionskarte.

**17.** Verwendung von Vektoren nach einen oder mehreren der Ansprüche 12 bis 16 zur Insertionsmutage – nese von Gram – positiven Bakterien.

**18.** Verwendung von Vektoren nach einem oder mehreren der Ansprüche 12 bis 16, zur Bestimmung des Wirtbereichs des in dem DNA – Segment (c) enthaltenen Replikons.

## Claims

**1.** A process for the conjugative transfer of mobilizable vectors from E.coli mobilizer strains into Gram – positive bacteria, characterised in that restriction defective cells of a Gram – positive bacterium are prepared and these are crossed over by processes known per se with an E.coli strain carrying the mobilizable vector.

**2.** A process according to Claim 1, characterised in that the vector contains a DNA region (Mob – site) and the E.coli mobilizer strain contains a plasmid integrated in the chromosome or in the free form, which is capable of providing the functions necessary for mobilization.

**3.** A process according to Claim 2, characterised in that the vector carries a DNA fragment (Mob – site) 1.9 kb in size of the plasmid RP4 and is integrated with the chromosome of the E.coli mobilizer strain of an RP4 derivative whose transfer function has an in – trans action on the mob – site of the said vector.

**4.** A process according to Claim 3, characterised in that the E.coli strain is selected from E.coli S17 – 1, E.coli SM10 and E.coli S68 – 7.

**5.** A process according to one or more of Claims 2 to 4, characterised in that the E.coli vector is selected from pSUP101, pSUP102, pSUP201, pSUP202, pSUP203, pSUP205, pSUP301 and pSUP401.

**6.** A process according to one or more of Claims 1 to 5, characterised in that the restriction system of the Gram – positive bacterium to be employed is impaired by a heat shock immediately before the crossing over.

**7.** A process according to one or more of Claims 1 to 6, characterised in that intact cells of the recipient are employed.

**8.** A process according to one or more of Claims 1 to 6, characterised in that sphaero – plasticized cells of the recipient are employed.

**9.** A process according to one or more of Claims 1 to 8, characterised in that there is employed a mobilizable vector into which is inserted a gene (fragment) which is active in a Gram positive bacterium and recombines homologously with the corresponding gene in the bacterium to be transformed.

**10.** A process according to one or more of Claims 1 to 8, characterised in that a mobilizable vector is employed in which is inserted a transposon and optionally a gene (fragment) which encodes for an enzyme activity in E.coli or a Gram positive bacterium.

**11.** A process according to one or more of Claims 1 to 8, characterised in that a mobilizable vector is employed which fuses with a vector or a plasmid replicating in the recipient to form a shuttle vector.

**12.** Mobilizable, non – autotransferable vector, composed of
a) a DNA segment containing a replicon which is functional in E.coli,
b) a second DNA segment, containing the DNA fragments (Mob – site and ori T) which encode for the mobilization and transfer function,
c) a third DNA segment derived from a vector which replicates in coryneform bacteria, in particular corynebacterium glutamicum, and
d) optionally a transposon instead of (c) or contained in (c).

**13.** A vector according to Claim 12, characterised in that the third DNA segment (c) is derived from the vector pCV35 shown in Figure 1.

**14.** A vector according to one or more of Claims 12 to 13, characterised in that the DNA segments (a) and (b) are derived from one of the vectors selected from pSUP101, pSUP102, pSUP201, pSUP202, pSUP203, pSUP205, pSUP301 and pSUP401.

**15.** Vector pECM1 (Shuttle – Vector) according to Claims 12 to 14, characterised by the restriction map shown in Figure 1 and deposited in E.coli S17 – 1, DSM 4982.

**16.** Vector pECM3 according to Claims 12 to 14, characterised by the restriction map shown in Figure 2.

**17.** The use of vectors according to one or more of Claims 12 to 16 for the insertion mutagenesis of Gram – positive bacteria.

**18.** The use of vectors according to one or more of Claims 12 to 16 for determining the host region of the replicon contained in the DNA segment (c).

**Revendications**

**1.** Procédé de transfert conjugué de vecteurs mobilisables à partir de souches mobilisatrices E. coli dans des bactéries Gram positives, caractérisé en ce qu'on prépare des cellules à défaut de restriction d'une bactérie Gram positive et qu'on les croise selon un procédé connu en soi avec une souche E. coli portant le vecteur mobilisable.

**2.** Procédé selon la revendication 1, caractérisé en ce que le vecteur possède une région d'ADN (site Mob) et la souche mobilisatrice E. coli contient un plasmide dans le chromosome sous forme intégrée ou libre, qui peut mettre à disposition les fonctions indispensables à la mobilisation.

**3.** Procédé selon la revendication 2, caractérisé en ce que le vecteur porte un fragment d'ADN de 1,9 kb (site Mob) du plasmide RP4 et que dans le chromosome de la souche mobilisatrice E. coli est intégré un dérivé RP4, dont la fonction de transfert s'exerce de manière intrans sur les sites Mob du vecteur cité.

**4.** Procédé selon la revendication 3, caractérisé en ce qu'on choisit la souche E. coli parmi le groupe E. coli S17 – 1, E. coli SM10 ou E. coli S68 – 7.

**5.** Procédé selon l'une ou plusieurs des revendications 2 à 4, caractérisé en ce qu'on choisit le vecteur E. coli parmi le groupe pSUP101, pSUP102, pSUP201, pSUP202, pSUP203, pSUP205, pSUP301, pSUP401.

**6.** Procédé selon l'une ou plusieurs des revebdications 1 à 5, caractérisé en ce qu'on endommage le système de restriction de la bactérie Gram positive à utiliser par un choc thermique immédiatement avant le croisement.

**7.** Procédé selon une ou plusieurs des revendications 1 à 6, caractérisé en ce qu'on utilise des cellules intactes du receveur.

**8.** Procédé selon une ou plusieurs des revendications 1 à 6, caractérisé en ce qu'on utilise des cellules sphéroplastifiées du receveur.

**9.** Procédé selon une ou plusieurs des revendications 1 à 8, caractérisé en ce qu'on utilise un vecteur mobilisable, dans lequel est inséré un gène (fragment) actif dans une bactérie Gram positive, qu'on recombine de manière homologue au gène correspondant dans la bactérie à transformer.

**10.** Procédé selon une ou plusieurs des revendications 1 à 8, caractérisé en ce qu'on utilise un vecteur mobilisable, dans lequel est inséré un transposon et le cas échéant un gène (fragment) codant pour une activité enzymatique dans E. coli ou dans une bactérie Gram positive.

**11.** Procédé selon une ou plusieurs des revendications 1 à 8, caractérisé en ce qu'on utilise un vecteur mobilisable qu'on fusionne avec un plasmide ou vecteur se répliquant dans le receveur pour donner un vecteur − navette.

**12.** Vecteur mobilisable, non autotransférable composé :
a) d'un segment d'ADN contenant un réplicon fonctionnel dans un E. coli,
b) d'un deuxième segment d'ADN contenant les fragments d'ADN codant pour la fonction de mobilisation et de transfert (site Mob et ori T),
c) d'un troisième segment d'ADN qui dérive d'un vecteur se répliquant dans les bactéries coryne − formes, notamment Corynebactérium glutamicum, et
d) qui contiennent éventuellement un transposon au lieu de (c) ou dans (c).

**13.** Vecteur selon la revendication 12, caractérisé en ce que le troisième segment d'ADN (c) dérive du vecteur pCV35, représenté à la figure 1.

**14.** Vecteur selon une ou plusieurs des revendications 12 à 13, caractérisé en ce que les segments d'ADN (a) et (b) dérivent d'un des vecteurs du groupe pSUP101, pSUP102, pSUP201, pSUP202, pSUP203, pSUP205, pSUP301 ou pSUP401.

**15.** Vecteur pECM1 (vecteur − navette) selon les revendications 12 à 14, caractérisé par la carte de restriction reproduite à la figure 1, déposée dans E. coli S17 − 1, DSM 4982.

**16.** Vecteur pECM3 selon les revendications 12 à 14, caractérisé par la carte de restriction reproduite à la figure 2.

**17.** Utilisation de vecteurs selon une ou plusieurs des revendications 12 à 16 pour la mutagénèse d'insertion de bactéries Gram positives.

**18.** Utilisation de vecteurs selon une ou plusieurs des revendications 12 à 16, pour déterminer la plage d'accueil du réplicon contenu dans le segment d'ADN (c).

Die Vektoren pCV35, pSUP102 und pECM1

Legende:

kan : DNA Sequenz, welche Resistenz gegen das Antibiotikum Kanamycin
vermittelt

cm : DNA Sequenz, welche Resistenz gegen das Antibiotikum Chloramphenicol
vermittelt

tet : DNA Sequenz, welche Resistenz gegen das Antibiotikum Tetracyclin
vermittelt

ori V : Startpunkt der vegetativen Replikation in E.coli

ori T : Startpunkt der transfer-Replikation

Der mobilisierbare *E.coli-C.glutamicum* shuttle-Vektor pECM3

Legende:

ori V: Startpunkt der vegetativen Replikation in *E.coli*
ori T: Startpunkt der Transfer-Replikation, Teil der mob-site
cm   : DNA Sequenz, welche Resistenz gegen das Antibiotikum
       Chloramphenicol vermittelt

Der pHM1519 Anteil des Vektors pECM3 erstreckt sich von Nukleotid 2200
bis Nukleotid 5100.

MUTATION OF CHROMOSOMAL DNA BY GENE DISRUPTION

chromosomal DNA mutated by plasmid integration

Abbreviations:
Cm[R]:        chloramphenicol acetyltransferase gene
Km[R]:        aminoglycoside phosphotransferase gene
lysA:        lysA gene coding for the meso-diaminopimelate-dehydrogenase
lysA-int:    internal fragment of the lysA gene lacking start and stop of
             the coding region
Δ lysA:      incomplete lysA genes after integration of pEMlys2 which are
             missing either beginning or end of the coding region

15

Konstruktion des Vektors pK18lys

Km : Kanamycin-Resistenzgen

lacZ-alpha: N-terminale Region des *lacZ*-Gens, das zur alpha-Komplementation befähigt

lysA : *lysA*-Gen

lysA-int : internes Fragment des *lysA*-Gens

oriV : Startpunkt der vegetativen Replikation in *E.coli*

Konstruktion des Vektors pEMlysl

Legende:

| | |
|---|---|
| Cm | : Chloramphenicol-Resistenzgen |
| Km | : Kanamycin-Resistenzgen |
| Tc | : Tetracyclin-Resistenzgen |
| lacZ-alpha: | N-terminale Region des *lacZ*-Gens, das zur alpha-Komplementation befähigt |
| lysA-int | : Internes Fragment des *lysA*-Gens |
| oriV | : Startpunkt der vegetativen Replikation in *E.coli* |
| oriT | : Startpunkt der Transferreplikation |

Konstruktion des Vektors pEMlys2

Amp      : Ampicillin-Resistenzgen
Cm       : Chloramphenicol-Resistenzgen
Km       : Kanamycin-Resistenzgen
lysA-int : internes Fragment des *lysA*-Gens
oriV     : Startpunkt der vegetativen Replikation in *E.coli*
oriT     : Startpunkt der Transferreplikation